# EUROPEAN PATENT SPECIFICATION

(11) **EP 1 885 339 B1**
(45) Date of publication and mention of the grant of the patent: **29.07.2015**
(21) Application number: 06769997.5
(22) Date of filing: 04.05.2006
(51) Int. Cl.: A61K 9/16, A61K 9/20, A61K 9/28, A61K 31/506

(54) **FORMULATIONS OF A SRC/ABL INHIBITOR**
FORMULIERUNGEN EINES SRC-/ABL-HEMMERS
FORMULATIONS D'INHIBITEUR DE SRC/ABL

(30) Priority: 05.05.2005 US 678030 P
(43) Date of publication of application: 13.02.2008
(73) Proprietor: Bristol-Myers Squibb Holdings Ireland, 6312 Steinhausen (CH)
(72) Inventor: GAO, Julia ZH, Plainsboro, New Jersey 08536 (US); MOTHERAM, Rajeshwar, Dayton, New Jersey 08810 (US)
(74) Representative: Beacham, Annabel Rose
(86) International application number: PCT/US2006/017073
(87) International publication number: WO 2006/121742

(56) References cited:
- WO-A2-2005/077945
- US-B1- 6 596 746

## Description

### FIELD OF THE INVENTION

Disclosed herein are pharmaceutical compositions of 'N-(2-Chloro-6-methylphenyl)-2-[[6-[4-(2-hydroxyethyl)-1-piperazinyl]-2-methyl-4-pyrimidinyl]amino]-5-thiazolecarboxamide, and such pharmaceutical compositions for use in the treatment of oncological and immunological disorders.

### BACKGROUND OF THE INVENTION

The compound of formula (I) 'N-(2-Chloro-6-methylphenyl)-2-[[6-[4-(2-hydroxyethyl)-1-piperazinyl]-2-methyl-4-pyrimidinyllamino]-5-thiazolecarboxamide, is a protein tyrosine kinase inhibitor, including Src Kinase, Bcr/Abl inhibitor, and is also known as a Src/Abl inhibitor and is useful in the treatment of oncological and immunologic diseases.

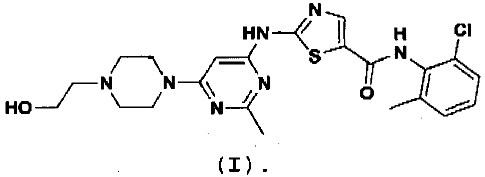

The compound of formula (I) and its preparation have been previously described in U.S. Patent No. 6,596,746, issued July 22, 2003. The use of the compound in the treatment of oncological and immunological disorders is described therein and in US Patent Publication No. US20040054186, published March 18, 2004. The compound is, in one embodiment, a crystalline monohydrate form such as described in U.S. Patent Application Serial No. 11/051,208, filed February 4, 2005. Alternatively, the compound of formula (I) may exist in other crystalline forms, either as a neat compound or as a solvate.

### SUMMARY OF THE INVENTION

Disclosed herein are pharmaceutical compositions of the compound of formula (I), and such compositions for use in treating immunologic and oncological disorders.

The pharmaceutical compositions comprise a pharmaceutically acceptable carrier and a therapeutically effective amount of the compound of formula (I), solvate, hydrate or pharmaceutically acceptable salt form thereof, and
a non-reactive coating, wherein the non-reactive coating is a coating having polyethylene glycol as plasticizer.

The non-reactive coating does not cause decomposition of the compound of formula (I). In another embodiment, the pharmaceutical composition having a non-reactive coating does not cause decomposition of the compound of formula (I) at varying concentrations of the compound of formula (I) and/or at high temperature and/or humidity.

In another aspect, the present disclosure is directed to pharmaceutical compositions having both intragrannular and extragranular microcrystalline cellulose.

Another embodiment provides the use of pharmaceutical compositions for the manufacture of a medicament for the treatment of oncological and immunological diseases.

The invention may be embodied in other specific forms without departing from the essential attributes thereof. This invention also encompasses all combinations of alternative aspects of the invention noted herein. It is understood that any and all embodiments of the present invention may be taken in conjunction with any other embodiment to describe additional embodiments of the present invention. Furthermore, any elements of an embodiment are meant to be combined with any and all other elements from any of the embodiments to describe additional embodiments.

### DETAILED DESCRIPTION OF EMBODIMENTS

One embodiment is directed to a pharmaceutical composition for oral administration comprising a pharmaceutically acceptable carrier and a therapeutically effective amount of a compound of formula (I) or solvate, hydrate, or pharmaceutically acceptable salt thereof, 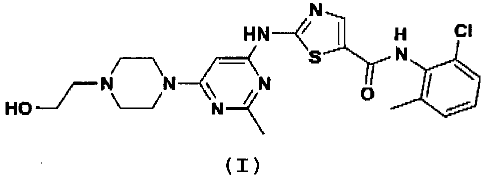 and a non-reactive coating, wherein the non-reactive coating is a coating having polyethylene glycol as plasticizer.

Another embodiment is directed to a pharmaceutical composition of the compound of formula (I) wherein the non-reactive coating does not react with the compound of formula (I).

Another embodiment is directed to a pharmaceutical composition of the compound of formula (I) wherein the pharmaceutically acceptable carrier comprises lactose monohydrate, microcrystalline cellulose, hydroxypropyl cellulose, croscarmellose sodium, and magnesium stearate.

Another embodiment is directed to a pharmaceutical composition of the compound of formula (I) wherein the microcrystalline cellulose is present in both intragranular and extragranular phase.

Another embodiment is directed to a pharmaceutical composition of the compound of formula (I) wherein about 15% by weight of the microcrystalline cellulose is extragranular.

The invention may be embodied in other forms without departing from the essential attributes thereof. This invention also encompasses all combinations of alternative aspects and embodiments of the invention noted herein. It is understood that any and all embodiments of the present invention may be taken in conjunction with any other embodiment to describe additional embodiments of the present invention. Furthermore, any elements of an embodiment are meant to be combined with any and all other elements from any of the embodiments to describe additional embodiments.

In general, the compound of formula (I) is a crystalline monohydrate and the range for the compound of formula (I) in the composition can vary from about 5 to 50% by weight. In another embodiment, the drug substance component will range from about 20 to 30% by weight in the composition.

Other components that may be used in the composition are described below. One component is lactose monohydrate, wherein the component can vary from about 0-45% by weight. Alternatively, the component is about 29-38% by weight. Alternatively, lactose monohydrate may be replaced with dicalcium carbonate or mannitol. Another component is microcrystalline cellulose or silicified microcrystalline cellulose which can vary from about 20 to about90% by weight. Alternatively, it may be present in the range of about 29-38% by weight. Another component is hydroxypropyl cellulose which can vary from about 1-5% by weight or pregelatinized starch which can vary from about 5-10% by weight. Alternatively, it may be present at about 3% or 5% by weight. Another component is croscarmellose sodium or sodium starch glycolate which can vary from about 2 to 8% by weight. Alternatively, it may be present at about 4% by weight. Another component is magnesium stearate which can vary from about 0.25 - 1% by weight or sodium stearyl fumarate which can vary from 0.5-2% by weight. Alternatively, it may be present at about 0.5% by weight for magnesium stearate or at about 1% by weight for sodium stearyl fumarate. Another component is sodium lauryl sulfate which can vary from 0-2% by weight. Alternately, it may be present at about 1% by weight.

In one embodiment, the composition is formed into tablets and is then coated with a non-reactive coating. The non-reactive coating is one which does not cause degradation of the compound of formula (I) within the tablet.

Some coatings have plasticizers present which may react with the compound of formula (I) producing unwanted impurities in the compositions. The compositions of the invention utilize coatings having the non-reactive plasticizer polyethylene glycol. Such coating is distributed under the name Opadry ® White, sold by Colorcon, containing hydroxypropylmethylcellulose, titanium dioxide, and polyethylene glycol.

Additionally, it has been found that the use of both extragranullar and intragrannular microcrystalline cellulose improves the compactibility of the pharmaceutical composition.

A formulation using larger particle size of the compound (I) (D90 ∼ 120 µm) was found to have poor compression properties when all of microcrystalline cellulose is added intragranularly. Conversely, the formulation using particle size of the compound (I) with D90 up to about 130 µm was found to have acceptable compression properties when portion of the microcrystalline cellulose is added in the extragranular phase. Alternatively, the particle size of the compound (I) may be up to or equal to 150 µm.

It was found that using about 15% by weight of the microcrystalline cellulose in the extragranular phase improved the compression properties of the composition.

Improved compression of the composition is obtained by adding about 10-20% by weight, alternatively, 15% by weight, of the microcrystalline cellulose extragrannularly. The remainder of the microcrystalline cellulose was added in the intragrannular phase. By doing this, the compression of the composition was improved.

Other features of the invention will become apparent in the course of the following descriptions of exemplary embodiments which are given for illustration of the invention and are not intended to be limiting thereof.

The term "improved compactibility properties" as used herein includes, but is not limited to, tablets with desired mechanical strength which can be manufactured reproducibly at reasonable compression speeds and forces.

The term "Nonreactive coating" as used herein includes, but is not limited to, coating ingredients which do not react with the compound of interest to form degradant(s) at any storage conditions.

The term "intragranular" as used herein includes, but is not limited to, the components added or the process steps prior to the drying of the granulation (for example, prior to step 3 of the process as described below).

The term "extragranular" as used herein includes, but is not limited to, the components added or the process steps after the drying of the water (for example, after step 3 of the process as described below).

All numbers expressing quantities of ingredients, properties such as molecular weight, reaction conditions, and so forth that are preceded by the word "about" are to be understood as only approximations so that slight variations above and below the stated number may be used to achieve substantially the same results as the stated number. Accordingly, unless indicated to the contrary, numerical parameters preceded by the word "about" are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

It is to be understood that each of the variously stated ranges is intended to be continuous so as to include each numerical parameter between the stated minimum and maximum value of each range. It is to be further understood that, while not intending to limit the applicability of the doctrine of equivalents to the scope of the claims, each numerical parameter should at least be construed in a manner consistent with the reported number of significant digits for each numerical parameter and by applying ordinary rounding techniques. It is to be even further understood that, while not intending to limit the applicability of the doctrine of equivalents to the scope of the claims, even though a number may be contained within a numerical range wherein at least one of the minimum and maximum numbers of the range is or is not preceded by the word "about", each numerical value contained within the range may or may not be preceded by the word "about". For Example, a range of about 1 to about 10 includes about 1, about 2, 2, about 3, 3, about 4, 4, about 5, 5, about 6, 6, about 7, 7, about 8, 8, about 9, 9, and about 10.

### Example I

| **Ingredient** | **% (w/w)** | **5 mg strength (mg)** |
|---|---|---|
| (I) | 5.0 | 5.0 |
| Lactose monohydrate | 44.0 | 44.0 |
| Microcrystalline cellulose | 43.5 | 43.5 |
| Hydroxypropyl cellulose | 3.0 | 3.0 |
| Croscarmellose sodium | 4.0 | 4.0 |
| Magnesium stearate | 0.5 | 0.5 |
| Opadry^{®} White | 4.0 | 4.0 |
| Total | - | 104.0 |

### Example II

| **Ingredient** | **% (w/w)** | **50 mg strength (mg)** |
|---|---|---|
| (I) | 50.0 | 50.0 |
| Lactose monohydrate | 21.0 | 21.0 |
| Microcrystalline cellulose | 21.0 | 21.0 |
| Hydroxypropyl cellulose | 3.0 | 3.0 |
| Croscarmellose sodium | 4.0 | 4.0 |
| Magnesium stearate | 1.0 | 1.0 |
| Opadry^{®} White | 4.0 | 4.0 |
| Total | - | 104.0 |

### Example III

| **Ingredient** | **% w/w** |
|---|---|
| (I) | 25.0 |
| Lactose monohydrate | 33.75 |
| Microcrystalline cellulose | 33.75 |
| Hydroxypropyl cellulose | 3.0 |
| Croscarmellose sodium | 4.0 |
| Magnesium stearate | 0.5 |
| Opadry^{®} White | 3 - 4 |
| Total | - |

Examples IV-VII

| **Ingredient** | **20-mg strength** | **50-mg strength** | **70-mg strength** | **150 mg strength** |
|---|---|---|---|---|
| (I) | 20.0 mg | 50.0 mg | 70.0 mg | 150.0 |
| Lactose monohydrate | 27.0 mg | 67.5 mg | 94.5 mg | 202.5 |
| Microcrystalline cellulose | 27.0 mg | 67.5 mg | 94.5 mg | 202.5 |
| Hydroxypropyl cellulose | 2.4 mg | 6.0 mg | 0.4 mg | 18.0 |
| Croscarmellose sodium | 3.2 mg | 8.0 mg | 11.2 mg | 24.0 |
| Magnesium stearate | 0.4 mg | 1.0 mg | 1.4 mg | 3.0 |
| Opadry® White | 3.2 mg | 7.0 mg | 8.4 mg | 18.0 |
| Total | 83.2 mg | 207.0 mg | 288.4 mg | 618.0 |

The above examples were prepared using the following procedure:
**1** Mix compound (I) as the crystalline monohydrate, lactose monohydrate, portion or all of microcrystalline cellulose, croscarmellose sodium, and hydroxypropyl cellulose in a suitable mixer.
**2** Granulate the blend from step [1] with water in a suitable mixer. (Intragrannular step)
**3** Dry the granulation from step [2].
**4** Pass the dried granulation from step [3] through an appropriate screen or mill.
**5** Add remaining portion of microcrystalline cellulose to the dried granulation from step [4] and blend in a suitable mixer. (Extragrannular step)
**6** Add prescreened magnesium stearate to the blend from step [5] and blend in a suitable mixer (Extragranular step).
**7** Compress the blend in step [6] into tablets.
**8** Prepare the film-coating suspension.
**9** Film-coat the tablets.

In one embodiment, the compositions of the present invention are useful for the treatment of cancers such as chronic myelogenous leukemia (CML), gastrointestinal stromal tumor (GIST), small cell lung cancer (SCLC), non-small cell lung cancer (NSCLC), ovarian cancer, melanoma, mastocytosis, germ cell tumors, acute myelogenous leukemia (AML), pediatric sarcomas, breast cancer, colorectal cancer, pancreatic cancer, prostate cancer, Philadelphia-chromosome positive acute lymphoblastic leukemia (Ph+ ALL) and others known to be associated with protein tyrosine kinases such as, for example, SRC, BCR-ABL and c-KIT. The compositions of the present invention are also useful in the treatment of cancers that are sensitive to and resistant to chemotherapeutic agents that target BCR-ABL and c-KIT, such as, for example, Gleevec^{®} (STI-571), and is useful in the treatment of patients resistant or intolerant to Gleevec ^{®} (STI-571) or AMN-107 for diseases such as chronic myelogenous leukemias (CML), or other cancers (including other leukemias) as described herein.

## Claims

1. A pharmaceutical composition for oral administration comprising a pharmaceutically acceptable carrier and a therapeutically effective amount of a compound of formula (I) solvate, hydrate, or pharmaceutically acceptable salt thereof 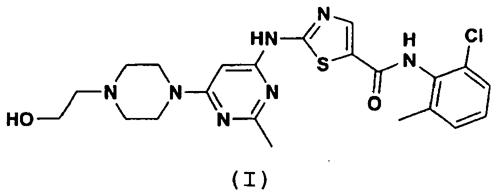 and a non-reactive coating wherein the non-reactive coating is a coating having polyethylene glycol as plasticizer.

2. The composition of claim 1 wherein the non-reactive coating does not react with the compound of formula (I).

3. The composition of claims 1-2, wherein the pharmaceutically acceptable carrier comprises lactose monohydrate, microcrystalline cellulose, hydroxypropyl cellulose, croscarmellose sodium, and magnesium stearate.

4. The composition of claim 3, wherein the microcrystalline cellulose is present in both intragranular and extragranular phase.

5. The composition of claims 1-3, wherein about 15% by weight of the microcrystalline cellulose is in extragranular phase.

## Patentansprüche

1. Pharmazeutische Zusammensetzung zur oralen Verabreichung, umfassend einen pharmazeutisch verträglichen Träger und eine therapeutisch wirksame Menge einer Verbindung der Formel (I), eines Solvats, Hydrats oder eines pharmazeutisch verträglichen Salzes davon 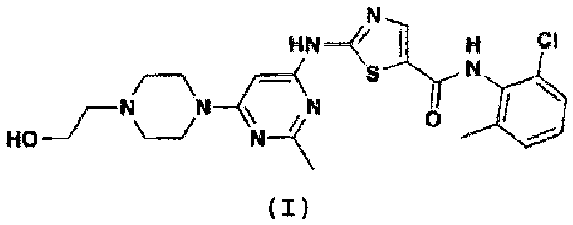 und eine nichtreaktive Beschichtung, wobei die nichtreaktive Beschichtung eine Beschichtung mit Polyethylenglykol als Weichmacher ist.

2. Zusammensetzung nach Anspruch 1, wobei die nichtreaktive Beschichtung nicht mit der Verbindung der Formel (I) reagiert.

3. Zusammensetzung nach Anspruch 1-2, wobei der pharmazeutisch verträgliche Träger Lactosemonohydrat, mikrokristalline Cellulose, Hydroxypropylcellulose, Croscarmellose-Natrium und Magnesiumstearat umfasst.

4. Zusammensetzung nach Anspruch 3, wobei die mikrokristalline Cellulose sowohl in intragranularer als auch in extragranularer Phase vorhanden ist.

5. Zusammensetzung nach Anspruch 1-3, wobei etwa 15 Gew.-% der mikrokristallinen Cellulose in extragranularer Phase vorliegen.

## Revendications

1. Composition pharmaceutique pour administration orale, comprenant un support pharmaceutiquement acceptable et une quantité thérapeutiquement efficace d'un composé de formule (I), d'un solvate, d'un hydrate ou d'un sel pharmaceutiquement acceptable de celui-ci 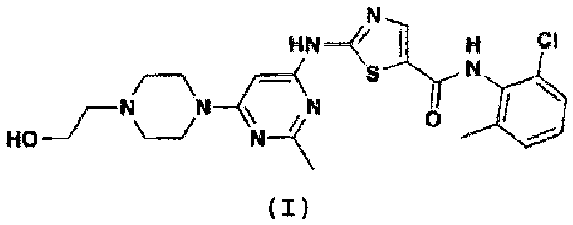 et un revêtement non réactif, où le revêtement non réactif est un revêtement présentant du polyéthylène glycol en tant que plastifiant.

2. Composition selon la revendication 1, dans laquelle le revêtement non réactif ne réagit pas avec le composé de formule (I).

3. Composition selon les revendications 1 à 2, dans laquelle le support pharmaceutiquement acceptable comprend du lactose monohydraté, de la cellulose microcristalline, de l'hydroxypropylcellulose, du croscarmellose sodique et du stéarate de magnésium.

4. Composition selon la revendication 3, dans laquelle la cellulose microcristalline est présente aussi bien en phase intragranulaire qu'en phase extragranulaire.

5. Composition selon les revendications 1 à 3, dans laquelle environ 15 % en poids de la cellulose microcristalline sont en phase extragranulaire.
